# EUROPÄISCHE PATENTSCHRIFT

(11) **EP 0 562 451 B1**
(45) Veröffentlichungstag und Bekanntmachung des Hinweises auf die Patenterteilung: **02.01.1997**
(21) Anmeldenummer: 93104423.4
(22) Anmeldetag: 18.03.1993
(51) Int. Cl.: C07C 31/125, C07C 69/80, C07C 29/17, C07C 45/50, C07C 45/74, C07C 47/02

(54) **Decylalkoholgemische, daraus erhältliche Phthalsäureester und ihre Verwendung als Weichmacher**
Mixtures of decyl alcohols, phthalic acid esters obtained from them and their application as plasticisers
Mélanges d'alcools décycliques, esters de l'acide phtalique obtenus de ceux-ci et leur utilisation comme agents plastifiants

(30) Priorität: 27.03.1992 DE 4210026
(43) Veröffentlichungstag der Anmeldung: 29.09.1993
(73) Patentinhaber: HOECHST AKTIENGESELLSCHAFT, 65926 Frankfurt am Main (DE)
(72) Erfinder: Bahrmann, Helmut, Dr. Dipl.-Chem., W-4236 Hamminkeln 3 (DE); Greb, Wolfgang, Dr. Dipl.-Ing., W-4220 Dinslaken (DE); Heymanns, Peter, Dr. Dipl.-Chem., W-4300 Essen 1 (DE); Lappe, Peter, Dr. Dipl.-Chem, W-4220 Dinslaken (DE); Müller, Thomas, Dipl.-Ing., W-4220 Dinslaken (DE); Szameitat, Jürgen, Dr. Dipl.-Chem., W-4230 Wesel (DE); Wiebus, Ernst, W-4200 Oberhausen 11 (DE)

(56) Entgegenhaltungen:
- EP-A- 0 052 999
- EP-A- 0 094 456
- EP-A- 0 157 316
- EP-A- 0 366 089
- DE-A- 2 627 354

## Beschreibung

Die Erfindung betrifft ein Verfahren zur Herstellung von Gemischen isomerer Decylalkohole.

Ester der Phthalsäure finden in großem Umfang als Weichmacher, insbesondere für Polyvinylchlorid, Verwendung. Als Alkoholkomponente werden vorwiegend primäre Alkohole mit 8 bis 10 Kohlenstoffatomen eingesetzt, größte Bedeutung unter ihnen hat gegenwärtig das 2-Ethylhexanol. Phthalsäureester kurzkettigerer Alkohole führen zu Weichmachern mit guter Gelierkraft. Nachteilig ist jedoch ihre höhere Flüchtigkeit. Längerkettige Ester gelieren dagegen langsamer und haben eine schlechtere Kältebeständigkeit.

Die Eigenschaften der Phthalsäureester-Weichmacher werden außer durch die Größe des Alkoholmoleküls auch durch die Verzweigung der Kohlstoffkette beeinflußt. So ergeben wenig verzweigte Alkohole Esterweichmacher hoher Kälteflexibilität. Weitgehend lineare Alkohole mit 8 bis 10 Kohlenstoffatomen im Molekül gewinnen daher als Alkoholkomponente zunehmend an Bedeutung. Voraussetzung für ihren Einsatz ist, daß sie in großen Mengen und kostengünstig zur Verfügung stehen.

Nach der deutschen Patentschrift 2 855 421 werden als Weichmacher Phthalate von C₉-Alkoholen eingesetzt, die durch Oxoreaktion von C₈-Olefinen, Hydrierung des Reaktionsproduktes und Veresterung der C₉-Alkohole mit Phthalsäureanhydrid erhalten werden. 3 bis 20 Gew.-% der Ausgangsolefine sollen in jeder Molekülkette ein Isobutanskelett, weniger als 3 Gew.-% der Olefine quaternären Kohlenstoff aufweisen und mehr als 90 Gew.-% der Gesamtmenge der Olefine als n-Octene, Monomethylheptene und Dimethylhexene vorliegen. Ferner soll das Gewichtsverhältnis der Gesamtmenge der n-Octene und Monomethylheptene zu den Dimethylhexenen mehr als 0,8 betragen.

Phthalsäureester auf Basis von C₁₀-Alkoholen sind Gegenstand der europäischen Patentanmeldung 03 66 089. Die C₁₀-Alkohole werden in Form eines Gemisches eingesetzt, das man durch Hydroformylierung einer Butenfraktion, Aldolkondensation des erhaltenen Aldehydgemisches und anschließende Hydrierung gewinnt. Der Hydroformylierungsschritt unterliegt nach der Verfahrensbeschreibung keinen Beschränkungen. So kann als Katalysator sowohl Kobalt als auch Rhodium eingesetzt werden, der Zusatz einer organischen Verbindung des dreiwertigen Phosphors wird nicht ausgeschlossen.

Ein anderer Weg zur Gewinnung von Didecylphthalatgemischen ist in der europäischen Patentanmeldung 04 24 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

Die bekannten Verfahren erfüllen in wirtschaftlicher und technischer Hinsicht noch nicht alle Anforderungen, die an einen im großen Maßstab ausgeübten Prozeß gestellt werden, sei es, daß die Ausgangsstoffe nicht in ausreichender Menge und/oder nicht kostengünstig zur Verfügung stehen oder daß die Umwandlung der Ausgangsstoffe in die Alkohole an zu aufwendige Prozesse gebunden ist. Bei Verfahren, die von Butenen ausgehen, die hydroformyliert werden, soll insbesondere der n-Valeraldehyd-Gehalt des Hydroformylierungsprodukts möglichst hoch sein, um die Bildung geradkettiger oder nur wenig verzweigter Alkohole zu fördern.

Es bestand daher die Aufgabe, ein Verfahren zu entwickeln, das nicht nur von Rohstoffen ausgeht, die preiswert zur Verfügung stehen, sondern die auch in technisch einfacher Weise in die gewünschten geradkettigen oder wenig verzweigten Alkohole überführt werden können.

Die Erfindung besteht in einem Verfahren zur Herstellung von Gemischen isomerer Decylalkohole durch Hydroformylierung von Buten-1 und Buten-2 enthaltenden Gemischen in zwei Stufen, wobei die Umsetzung in der ersten Stufe in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa und in der zweiten Stufe in homogener Phase in Gegenwart von Rhodiumverbindungen als Katalysatoren bei Temperaturen von 90 bis 180°C und Drücken von 10 bis 35 MPa zu Aldehydgemischen erfolgt, Abtrennung und Vereinigung der erhaltenen Aldehydgemische aus den Hydroformylierungsstufen, Kondensation des vereinigten Aldehydgemischs unter Bildung eines Aldolgemischs und Abtrennung und Hydrierung des Aldolgemischs zu einem Gemisch isomerer Decylalkohole.

Die zur Herstellung des Gemischs isomerer Decylalkohole eingesetzten Buten-1 und Buten-2 enthaltenden Gemische fallen als Raffinerienebenprodukte bei der Herstellung von Automobiltreibstoffen und bei der Herstellung von Ethylen durch thermische Spaltung höherer Kohlenwasserstoffe zwangsweise in erheblichen Mengen an. Man gewinnt sie aus den C₄-Crackschnitten des Pyrolyseproduktes durch Extraktion des Butadiens mit einem selektiven Lösungsmittel und anschließende Abtrennung des Isobutens vorzugsweise durch Umwandlung in Methyl-tert.butylether. Das von Butadien befreite Pyrolyseprodukt wird als Raffinat I bezeichnet, ist darüberhinaus auch noch das Isobuten abgetrennt, spricht man von Raffinat II. Statt das Butadien zu extrahieren, kann es auch im C₄-Crackschnitt partiell zu Butenen hydriert werden. Nach Abtrennung des i-Butens erhält man ein Buten-1/Buten-2-Gemisch, das besonders geeignet zur Weiterverarbeitung auf C₁₀-Alkohole ist. Schließlich geht man in letzter Zeit auch dazu über, das abgetrennte Butadien zu Butan zu hydrieren und in die Spaltung zurückzuführen um die Ethylen- und Propylenausbeute zu erhöhen.

Erfindungsgemäß werden Buten-1 und Buten-2 enthaltende Gemische z.B. in Form des Raffinats II, aber auch anderer Herkunft und Zusammensetzung in zwei Stufen hydroformyliert. In der ersten Stufe erfolgt bevorzugt die Umsetzung des Buten-1 zu einem Gemisch, das vorwiegend aus n-Valeraldehyd und, in untergeordneter Menge, aus i-Valeraldehyd besteht. Die Reaktion verläuft unter Bedingungen, die eine Isomerisierung des Buten-1 zu Buten-2 weitgehend ausschließen. In der zweiten Stufe wird das Buten-2 zu einem Gemisch von n-Valeraldehyd und i-Valeraldehyd hydroformyliert.

Die erste Stufe der Hydroformylierung führt man als heterogene Reaktion in einem Zweiphasensystem durch, eine Umsetzung, die z.B. in der DE-PS 26 27 354 beschrieben ist. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist bekannt, vgl. z.B. DE-PS 26 27 354 und DD-PS 259 194. Die Reaktion der Butene erfolgt bei Temperaturen von 70 bis 150°C, vorzugsweise 100 bis 130°C und unter Drücken im Bereich von 0,4 bis 30, insbesondere 1 bis 10 MPa mit Wassergas, das Kohlenmonoxid und Wasserstoff im Volumenverhältnis 1 : 10 bis 10 : 1 enthält. Die Rhodiumkonzentration beträgt 20 bis 1000 Gew.-ppm, vorzugsweise 50 bis 500 Gew.-ppm, bezogen auf die wäßrige Katalysatorlösung, je mol Rhodium setzt man 4 bis 100 mol wasserlösliches Phosphin ein. Das Volumverhältnis von wäßriger zu organischer Phase beträgt 0,1 bis 10 : 1.

Der Butenumsatz wird deutlich erhöht, wenn man der wäßrigen Katalysatorlösung ein Phasentransferreagenz (Lösungsvermittler) zusetzt. Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 4 Kohlenstoffatomen bedeuten und E insbesondere für Sulfat, Tetrafluorborat, Acetat, Methosulfat, Benzolsulfonat, Alkylbenzolsulfonat, Toluolsulfonat, Lactat oder Citrat steht.

Das Aldehydgemisch enthält bei einem Buten-1-Umsatz, der je nach den gewählten Reaktionsparametern bis zu 95 % beträgt, 90 und mehr Prozent n-Valeraldehyd, der Rest ist i-Valeraldehyd.

In der ersten Stufe nicht umgesetztes Olefin, vorwiegend Buten-2, wird in einer zweiten Reaktionsstufe in homogener Phase und in Gegenwart von Rhodium als Katalysator hydroformyliert. Als Reaktionstemperaturen haben sich 90 bis 180°C und vorzugsweise 130 bis 150°C und Drücke von 10 bis 35 MPa, insbesondere von 20 bis 30 MPa bewährt. Rhodium wird dem Reaktionsgemisch als Metall, zweckmäßig in feinverteilter Form oder, besser, als in organischen Medien lösliche Verbindung zugeführt, z.B. als Carbonylverbindung oder als Salz einer Carbonsäure. Die Rhodiumkonzentration beträgt 2 bis 100 Gew.-ppm, vorzugsweise 5 bis 30 Gew.-ppm, bezogen auf die in die zweite Reaktionsstufe eingeleiteten Butene. Die Anwesenheit eines Lösungsmittels wie Toluol, Xylol oder Tetrahydrofuran ist nicht unbedingt erforderlich, weil seine Funktion vom Einsatzstoff und vom Reaktionsprodukt übernommen werden kann. Das Wassergas hat die gleiche Zusammensetzung wie im ersten Reaktionsschritt. In Abhängigkeit von den Reaktionsbedingungen werden bis zu 99 % des eingesetzen Olefins in n- und i-Valeraldehyd überführt.

Nach Beendigung der Hydroformylierung wird das Aldehydgemisch beider Reaktionsschritte vom Katalysator, von den nichtumgesetzten Reaktionsteilnehmern und den übrigen Reaktionsprodukten abgetrennt. Das geschieht im Falle der heterogenen Reaktion (erste Stufe) durch einfache Phasentrennung. Bei Umsetzung in homogener Phase, d.h. der zweiten Stufe des neuen Prozesses, ist die Destillation das übliche Trennverfahren.

Die Aldolkondensation der als Gemisch vorliegenden Aldehyde erfolgt auf konventionellem Wege unter der Einwirkung basischer Katalysatoren. Eine Vorbehandlung der Aldehyde, z.B. eine spezielle Reinigung, ist nicht erforderlich. Als Katalysatoren finden Alkalicarbonate oder Alkalihydroxide, insbesondere Verbindungen des Natriums oder Kaliums und Amine, vorzugsweise tertiäre Amine, wie Triethylamin, Tri-n-propylamin, Tri-n-butylamin, Anwendung. Man arbeitet bei Temperaturen von 60 bis 160°C, insbesondere 80 bis 130°C und bei Normaldruck oder bei bis etwa 1 MPa erhöhtem Druck. Die Reaktionszeit beträgt wenige Minuten bis zu mehreren Stunden und ist insbesondere abhängig von Katalysatortyp und Reaktionstemperatur. Auf Grund seiner höheren Reaktionsgeschwindigkeit dimerisiert vornehmlich n-Valeraldehyd mit sich selbst oder mit isomeren Valeraldehyden zu Decenalen, eine Kondensation der verzweigten C₅-Aldehyde untereinander tritt dagegen völlig in den Hintergrund.

Das durch Kondensation erhaltene Aldehydgemisch wird anschließend zu einem Decylalkoholgemisch hydriert. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung wird das Decylalkoholgemisch destilliert. Es eignet sich vorzüglich als Alkoholkomponente in Phthalsäureestern, die als Weichmacher verwendet werden sollen. Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem Decylalkoholgemisch im Molverhältnis 1 : 2 in einer Stufe um. Die Reaktionsgeschwindigkeit kann durch Katalysatoren und/oder durch Erhöhung der Reaktionstemperatur erhöht werden. Um das Gleichgewicht in Richtung der Esterbildung zu verschieben, ist es erforderlich, das gebildete Wasser aus dem Reaktionsgemisch zu entfernen.

Die aus dem erfindungsgemäßen Decylalkoholgemisch erhaltenen Phthalate zeichnen sich als Weichmacher durch hervorragende Kälteeigenschaften aus.

## Patentansprüche

1. Verfahren zur Herstellung von Gemischen isomerer Decylakohole, erhalten durch Hydroformylierung von Buten-1 und Buten-2 enthaltenden Gemischen in zwei Stufen, wobei die Umsetzung in der ersten Stufe in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa und in der zweiten Stufe in homogener Phase in Gegenwart von Rhodiumverbindungen als Katalysatoren bei Temperaturen von 90 bis 180°C und Drücken von 10 bis 35 MPa zu Aldehydgemischen erfolgte, Abtrennung und Vereinigung der erhaltenen Aldehydgemische aus den Hydroformylierungsstufen, Kondensation des vereinigten Aldehydgemischs unter Bildung eines Aldolgemischs und Abtrennung und Hydrierung des Aldolgemischs zu einem Gemisch isomerer Decylalkohole.

2. Verfahren zur Herstellung von Gemischen isomerer Decylakohole nach Anspruch 1, dadurch gekennzeichnet, daß die Umsetzung in der ersten Hydroformylierungsstufe bei Temperaturen von 100 bis 130°C und unter Drücken von 1 bis 10 MPa erfolgt.

3. Verfahren zur Herstellung von Gemischen isomerer Decylakohole nach Anspruch 1 oder 2, dadurch gekennzeichnet, daß die Umsetzung in der ersten Hydroformylierungsstufe bei einer Rhodiumkonzentration von 20 bis 1000 Gew.-ppm. vorzugsweise 50 bis 500 Gew.-ppm, bezogen auf die wäßrige Katalysatorlösung, erfolgt.

4. Verfahren zur Herstellung von Gemischen isomerer Decylalkohole nach einem oder mehreren der Ansprüche 1 bis 3, dadurch gekennzeichnet, daß in der ersten Hydroformylierungsstufe je mol Rhodium 4 bis 100 mol wasserlösliches Phosphin eingesetzt werden.

5. Verfahren zur Herstellung von Gemischen isomerer Decylalkohole nach einem oder mehreren der Ansprüche 1 bis 4, dadurch gekennzeichnet, daß in der ersten Hydroformylierungsstufe der wäßrigen Katalysatorlösung ein Phasentransferreagenz zugesetzt wird.

6. Verfahren zur Herstellung von Gemischen isomerer Decylalkohole nach einem oder mehreren der Ansprüche 1 bis 5, dadurch gekennzeichnet, daß die Umsetzung in der zweiten Hydroformylierungsstufe bei 130 bis 150°C und Drücken von 20 bis 30 MPa erfolgt.

7. Verfahren zur Herstellung von Gemischen isomerer Decylalkohole nach einem oder mehreren der Ansprüche 1 bis 6, dadurch gekennzeichnet, daß die Umsetzung in der zweiten Hydroformylierungsstufe bei einer Rhodiumkonzentration von 2 bis 100 Gew.-ppm, vorzugsweise 5 bis 30 Gew.-ppm, bezogen auf die in die zweite Hydroformylierungsstufe eingeleiteten Butene, erfolgt.

## Claims

1. A process for the preparation of a mixture of isomeric decyl alcohols, obtained by hydroformylation of 1-butene- and 2-butene-containing mixtures in two stages, the reaction in the first stage being carried out in a heterogeneous reaction system with the use of rhodium compounds containing complexed water-soluble phosphines as catalysts at temperatures of 70 to 150°C and pressures of 0.4 to 30 MPa and the reaction in the second stage being carried out in homogeneous Phase in the presence of rhodium compounds as catalysts at temperatures of 90 to 180°C and pressures of 10 to 35 MPa to give aldehyde mixtures, isolation and combination of the resulting aldehyde mixtures from the hydroformylation stages, condensation of the combined aldehyde mixture with formation of an aldol mixture and isolation and hydrogenation of the aldol mixture to give a mixture of isomeric decyl alcohols.

2. A process for the preparation of the mixture of isomeric decyl alcohols as claimed in claim 1, wherein the reaction in the first hydroformylation stage is carried out at temperatures of 100 to 130°C and at pressures of 1 to 10 MPa.

3. A process for the Preparation of the mixture of isomeric decyl alcohols as claimed in claim 1 or 2, wherein the reaction in the first hydroformylation stage is carried out at a rhodium concentration of 20 to 1000 ppm by weight, preferably 50 to 500 ppm by weight, based on the aqueous catalyst solution.

4. A process for the preparation of the mixture of isomeric decyl alcohols as claimed in one or more of claims 1 to 3, wherein, in the first hydroformylation stage, 4 to 100 mol of water-soluble phosphine are used per mole of rhodium.

5. A process for the preparation of the mixture of isomeric decyl alcohols as claimed in one or more of claims 1 to 4, wherein, in the first hydroformylation stage, a phase transfer reagent is added to the aqueous catalyst solution.

6. A process for the preparation of the mixture of isomeric decyl alcohols as claimed in one or more of claims 1 to 5, wherein the reaction in the second hydroformylation stage is carried out at 130 to 150°C and pressures of 20 to 30 MPa.

7. A process for the preparation of the mixture of isomeric decyl alcohols as claimed in one or more of claims 1 to 6, wherein the reaction in the second hydroformylation stage is carried out at a rhodium concentration of 2 to 100 ppm by weight, preferably 5 to 30 ppm by weight, based on the butenes introduced into the second hydroformylation stage.

## Revendications

1. Procédé de préparation de mélanges d'alcools décyliques isomères obtenus par hydroformylation de mélanges contenant du butène-1 et du butène-2 en deux stades opératoires, la réaction étant réalisée au premier stade en système hétérogène avec utilisation en tant que catalyseurs de dérivés du rhodium contenant des phosphines solubles dans l'eau en liaison complexe, à des températures de 70 à 150°C et des pressions de 0,4 à 30 MPa et au deuxième stade en phase homogène en présence de dérivés du rhodium qui servent de catalyseurs, à des températures de 90 à 180°C et des pressions de 10 à 35 MPa, ces opérations donnant des mélanges d'aldéhydes qu'on sépare, on combine les mélanges d'aldéhydes obtenus aux opérations d'hydroformylation, on condense le mélange d'aldéhydes combiné avec formation d'un mélange d'aldols qu'on sépare et qu'on hydrogène en un mélange d'alcools décyliques isomères.

2. Procédé de préparation de mélanges d'alcools décyliques isomères selon revendication 1, caractérisé en ce que la réaction, au premier stade d'hydroformylation, est réalisée à des températures de 100 à 130°C et des pressions de 1 à 10 MPa.

3. Procédé de préparation de mélanges d'alcools décyliques isomères selon revendication 1 ou 2, caractérisé en ce que la réaction du premier stade d'hydroformylation est réalisée avec une concentration en rhodium de 20 à 1 000 ppm en poids, de préférence de 50 à 500 ppm en poids, par rapport à la solution aqueuse de catalyseur.

4. Procédé de préparation de mélanges d'alcools décyliques isomères selon une ou plusieurs des revendications 1 à 3, caractérisé en ce que, au premier stade d'hydroformylation, on utilise de 4 à 100 mol de phosphine soluble dans l'eau par mole de rhodium.

5. Procédé de préparation de mélanges d'alcools décyliques isomères selon une ou plusieurs des revendications 1 à 4, caractérisé en ce que, au premier stade d'hydroformylation, on ajoute un réactif à transfert de phase à la solution aqueuse du catalyseur.

6. Procédé de préparation de mélanges d'alcools décyliques isomères selon une ou plusieurs des revendications 1 à 5, caractérisé en ce que, au deuxième stade d'hydroformylation, la réaction est réalisée à des températures de 130 à 150°C et des pressions de 20 à 30 MPa.

7. Procédé de préparation de mélanges d'alcools décyliques isomères selon une ou plusieurs des revendications 1 à 6, caractérisé en ce que, au deuxième stade d'hydroformylation, la réaction est réalisée avec une concentration en rhodium de 2 à 100 ppm en poids, de préférence de 5 à 30 ppm en poids, par rapport aux butènes envoyés au deuxième stade d'hydroformylation.
